Europäisches Patentamt

European Patent Office

Office européen des brevets

(11) Veröffentlichungsnummer : **0 037 890**
**B1**

(12) **EUROPÄISCHE PATENTSCHRIFT**

(45) Veröffentlichungstag der Patentschrift :
18.01.84

(51) Int. Cl.³ : **C 07 C 89/02**, C 07 C 91/10

(21) Anmeldenummer : 81101251.7

(22) Anmeldetag : 21.02.81

(54) Verfahren zur Herstellung von 1-Amino-propandiol-(2,3) (I).

(30) Priorität : 12.04.80 DE 3014098

(43) Veröffentlichungstag der Anmeldung :
21.10.81 Patentblatt 81/42

(45) Bekanntmachung des Hinweises auf die Patenterteilung : 18.01.84 Patentblatt 84/03

(84) Benannte Vertragsstaaten :
BE CH DE FR GB LI NL

(56) Entgegenhaltungen :
DE-C- 694 992
JOURNAL OF ORGANIC CHEMISTRY, Band 27, Nr. 6, 12. Juni 1962, Seiten 2231-2233, New York, U.S.A. K. BAUM et al.: "The Mannich condensation of 3-amino-1,2-propanediol with 2,2-dinitropropanol and the nitration of the product"

(73) Patentinhaber : **Degussa Aktiengesellschaft**
**Weissfrauenstrasse 9**
**D-6000 Frankfurt am Main 1 (DE)**

(72) Erfinder : **Kleemann, Axel, Dr.**
**Greifenhagenstrasse 25**
**D-6450 Hanau 9 (DE)**
Erfinder : **Nygren, Robert, Dr.**
**Fürstenbergstrasse 8**
**D-6450 Hanau 9 (DE)**
Erfinder : **Wagner, Rudolf, Dr.**
**Greifenhagenstrasse 9**
**D-6450 Hanau 9 (DE)**

## Verfahren zur Herstellung von 1-Amino-propandiol-(2,3) (I)

Die Herstellung von 1-Amino-propandiol-(2,3) durch Addition von Ammoniak an Glycid wurde erstmals von L. Knorr u. E. Knorr (Ber. deutsch. Chem. Ges. 32, 750 (1899) beschrieben. Die Autoren setzten hierbei einen Gewichtsteil Glycid mit 100 Gewichtsteilen 25 %igem wässrigem Ammoniak ein und erhielten dann nach destillativer Aufarbeitung 1-Amino-propandiol-(2,3) in einer Ausbeute von 44 %, bezogen auf eingesetztes Glycid. Das Gewichtsverhältnis Glycid zu wässrigem Ammoniak (25 %ig) = 1 : 100 bedeutet ein Molverhältnis Glycid zu Ammoniak = 1 : 109.

Diese Herstellmethode von 1-Amino-propandiol-(2,3) wurde von K. Baum und W. T. Maurice (J. Org. Chem. 27, 2231 (1962) überprüft, wobei dann bei denselben Bedingungen eine Ausbeute von 68 % der Theorie erzielt werden konnte. Diese bessere Ausbeute findet ihre Begründung darin, dass die erstgenannten Autoren das Reaktionsprodukt bei 235-250 °C/320 mm Hg destillierten, während die zuletzt genannten Autoren schonender, nämlich bei 80-106 °C/0,1-0,15 mm Hg destillierten und somit keine Verluste durch thermische Zersetzung verursachten.

Wenn auch das zuletzt genannte Verfahren bessere Ausbeuten gegenüber dem Verfahren von Knorr (loc. cit.) bringt, so stellen die im Kreis zu führenden Mengen an wässrigem Ammoniak bei der technischen Durchführung eine erhebliche Belastung dar.

Ausserdem wird durch sie ein sehr grosser Reaktionsraum nötig auf Grund des oben genannten Molverhältnisses von Glycid zu Ammoniak, sowie eine Destillationsanlage zum Konzentrieren der im Kreis zu führenden verdünnten wässrigen Ammoniaklösung.

Wenn es auch nach der US-PS 3,544,632 bekannt ist, Alkanolamine durch Umsetzung von Alkylenoxiden mit Kohlenwasserstoffen und flüssigem Ammoniak in wässrigem Medium umzusetzen, so handelt es sich hier um die Reaktion von monofunktionellen Epoxiden mit Ammoniak, die nicht zur Selbstkondensation neigen.

Zwar wird ein diskontinuierliches Verfahren zur Herstellung von Aminodiolen in der FR-PS 1 423 428 beschrieben, bei denen aliphatische Epoxyalkohole eingesetzt werden. Glycid als Anfangsglied wird aber nicht genannt. Das Verfahren lässt sich auch nur diskontinuierlich durchführen und ist langwierig.

Zweck des erfindungsgemässen Verfahrens ist daher ein Verfahren zur Herstellung von 1-Amino-propandiol-(2,3) in guten Ausbeuten und auf technisch einfache Weise.

Es wurde nun gefunden, dass man die Umsetzung von Glycid mit Ammoniak in homogener flüssiger Phase mit guten Ausbeuten und ohne besonderem technischen Aufwand durchführen kann, wenn Glycid und flüssiges Ammoniak unter einem solchen Druck, dass das Ammoniak flüssig bleibt, miteinander umgesetzt werden.

Im allgemeinen liegt das Molverhältnis von Glycid zu flüssigem Ammoniak im Bereich von 1 : 5 bis 15.

Bevorzugt ist ein Molverhältnis von 1 : 15.

Molverhältnisse unter 5 : 1 sind zwar möglich ; die hierbei erhaltenen Ausbeuten an dem Aminopropandiol sind aber durch die erhöhte Bildung des Bis-Produktes verringert.

Oberhalb eines Molverhältnisses von 15 : 1 treten keine wesentlichen Ausbeutesteigerungen mehr ein, jedoch sinken dann die Raum-Zeitausbeuten.

Da das erfindungsgemässe Verfahren ohne Anwesenheit eines Lösungsmittels — wie bisher üblich — arbeitet, wird die notwendige Rezyklierung des flüssigen Ammoniaks sehr erleichtert. Ausserdem fallen Aufbereitungsanlagen für wässriges Ammoniak wie bei dem Verfahren von Baum und Maurice (loc. cit.) fort.

Gleichzeitig erhöht sich die Raum-Zeit-Ausbeute gegenüber den bisher bekannten Verfahren beträchtlich ; sie beträgt bei den oben genannten Verfahren (loc. cit.) 360 Ltr./kg · Std., während nach dem erfindungsgemässen Verfahren diese um den Faktor 60 erhöht werden konnte und somit zur Herstellung von 1 kg Aminopropandiol pro Stunde nur noch ein Reaktor von 6 Ltr. Inhalt benötigt wird.

Der Druckbereich liegt zwischen 8 bis 150 bar, bevorzugt bei 20 bis 90 bar.

Die jetzt im Vergleich zu Baum und Maurice (loc. cit.) erzielten Ausbeuten- und zwar in Abwesenheit eines Lösungsmittels, d. h. Verdünnungsmittels — sind insofern überraschend, da Glycid als bifunktionelle Verbindung zur Eigenkondensation neigt. Man hätte daher im Gegenteil erwarten müssen, dass in Abwesenheit eines Lösungsmittels die Ausbeute infolge Eigenkondensation stark absinkt.

Die Reaktionstemperaturen liegen bei 20 bis 180 °C, bevorzugt bei 50 bis 120 °C.

1-Aminopropandiol-(2,3) ist ein technisch interessantes Produkt für die Herstellung von Röntgenkontrastmittel, s. BE-PS 855 580, von entzündungshemmenden Mitteln, s. DE-OS 23 39 788, von Mitteln gegen Geflügelkrankheiten, s. US-PS 3 860 703, für Analgetika, s. GB-PS 1 672 359 und Kosmetika, s. DE-OS 24 21 618.

Die Anmeldung wird anhand der folgenden Beispiele näher erläutert :

Versuchsapparatur

Die nachstehenden Beispiele wurden in folgender Versuchsapparatur durchgeführt :

Aus einer mit Glycid gefüllten Vorlage und einer Druckflasche mit flüssigem Ammoniak wurden mittels zweier Pumpen mengengeregelt die Reaktanten in den Reaktor gefördert. Dieser bestand aus einem Doppelmantelrohr, wobei der äussere Mantelraum dazu diente, mit Hilfe von Wasser das Reaktionsgemisch im inneren Rohr auf die gewünschte Temperatur zu bringen und die Reaktionswärme abzuführen. Die Reaktion wurde in flüssiger, homogener Phase durchgeführt. Der zur Verflüssigung der Reaktanten nötige Druck wurde durch ein Druckregelventil am Ende des Doppelmantelrohres gehalten. Das innere Rohr, also die Reaktionszone, hatte ein Volumen von 4,2 Ltr. Nach Durchlaufen der Reaktionsstrecke wurde das Reaktionsgemisch am Druckregelventil entspannt und in eine Vorlage geleitet. Hierbei entwich aus dem Rohprodukt zu über 99 % das im Überschuss eingesetzte Ammoniak, welches nach Kondensation wieder der Reaktion zugeführt werden konnte. Aus dem Rohprodukt wurde durch fraktionierte Vakuumdestillation das 1-Amino-propandiol isoliert.

Beispiel 1

In den oben genannten Reaktor wurden bei 85 °C und 45 bar pro Stunde 0,65 kg Glycid und 2,23 kg flüssiges Ammoniak eindosiert. (Molverhältnis Glycid : Ammoniak = 1 : 15) Nach destillativer Aufarbeitung des Reaktionsproduktes erhielt man 0,55 kg Aminopropandiol pro Stunde, entsprechend 69 % d. Th., bezogen auf eingesetztes Glycid, Siedepunkt : 94 °C (0,2 Torr), Reinheit ≧ 99,5 % (Amintitration).

Beispiel 2

In den oben genannten Reaktor wurden bei 70 °C und 35 bar pro Stunde 0,53 kg Glycid und 1,25 kg flüssiges Ammoniak eindosiert. (Molverhältnis Glycid : Ammoniak = 1 : 10,1) Nach destillativer Aufarbeitung des Reaktionsproduktes erhielt man 0,4 kg Aminopropandiol pro Stunde, entsprechend 62 % der Theorie, bezogen auf eingesetztes Glycid.

Beispiel 3

In den oben genannten Reaktor wurden bei 80 °C und 40 bar pro Stunde 1,2 kg Glycid und 1,3 kg flüssiges Ammoniak eindosiert. (Molverhältnis Glycid : Ammoniak = 1 : 5) Nach destillativer Aufarbeitung des Reaktionsproduktes erhielt man 0,77 kg Aminopropandiol pro Stunde entsprechend 52 % der Theorie, bezogen auf eingesetztes Glycid.

Beispiel 4

In den oben genannten Reaktor wurden bei 60 °C und 25 bar pro Stunde 0,3 kg Glycid und 1,0 kg flüssiges Ammoniak eindosiert. (Molverhältnis Glycid : Ammoniak = 1 : 14,8) Nach destillativer Aufarbeitung des Reaktionsproduktes erhielt man 0,25 kg Aminopropandiol pro Stunde, entsprechend 69 % der Theorie, bezogen auf eingesetztes Glycid.

Auch in den Beispielen 2-4 entsprachen die Siedepunkte und die Reinheit des Produktes den Angaben in Beispiel 1.

### Ansprüche

1. Verfahren zur Herstellung von 1-Amino-propandiol-(2,3) durch Umsetzung von Glycid mit Ammoniak, dadurch gekennzeichnet, dass man Glycid und flüssiges Ammoniak unter einem solchen Druck miteinander umsetzt, dass das Ammoniak flüssig bleibt.

2. Verfahren nach Anspruch 1, dadurch gekennzeichnet, dass man Glycid und flüssiges Ammoniak in einem Molverhältnis von 1 : 5 bis 15 einsetzt.

3. Verfahren nach Anspruch 1, dadurch gekennzeichnet, dass man Glycid und flüssiges Ammoniak im Molverhältnis 1 : 15 einsetzt.

4. Verfahren nach Anspruch 1-3, dadurch gekennzeichnet, dass man die Reaktion unter einem Druck von 8 bis 150 bar, bevorzugt von 20 bis 90 bar, durchführt.

### Claims

1. A process for the production of 1-amino-propanediol-(2,3) by the reaction of glycidol with ammonia, characterised in that glycidol and liquid ammonia are reacted together under such a pressure that the ammonia remains liquid.

2. A process according to claim 1, characterised in that glycidol and liquid ammonia are used in a molar ratio of from 1 : 5 to 15.

3. A process according to claim 1, characterised in that glycidol and liquid ammonia are used in a molar ratio of 1 : 15.

4. A process according to claims 1 to 3, characterised in that the reaction is carried out under a pressure of from 8 to 150 bars, preferably from 20 to 90 bars.

### Revendications

1. Procédé de fabrication du 1-amino-propanediol-(2,3) par réaction du glycidol sur l'ammoniac, procédé caractérisé en ce que l'on fait réagir le glycidol et l'ammoniac liquide l'un sur l'autre sous une pression telle, que l'ammoniac reste liquide.

2. Procédé selon la revendication 1, caractérisé en ce que l'on met en œuvre le glycidol et l'ammoniac liquide dans un rapport molaire de 1 : 5 à 1 : 15.

3. Procédé selon la revendication 1, caractérisé en ce que l'on met en œuvre le glycidol et l'ammoniac liquide dans un rapport molaire de 1 : 15.

4. Procédé selon l'une quelconque des revendications 1 à 3, caractérisé en ce que l'on effectue la réaction sous une pression de 8 à 150 bars, et particulièrement 20 à 90 bars.